# EUROPÄISCHE PATENTSCHRIFT

(11) **EP 0 768 304 B1**
(45) Veröffentlichungstag und Bekanntmachung des Hinweises auf die Patenterteilung: **22.05.2002**
(21) Anmeldenummer: 96116409.2
(22) Anmeldetag: 14.10.1996
(51) Int. Cl.: C07D 239/46, A61K 31/305

(54) **N-(Pyrimidinyl)-benzolsulfonamide als Arzneimittel**
N-(Pyrimidinyl)-benzenesulfonamides as medicaments
N-(Pyrimidinyl)-benzènesulfonamides comme médicaments

(30) Priorität: 12.10.1995 CH 289395
(43) Veröffentlichungstag der Anmeldung: 16.04.1997
(73) Patentinhaber: F. HOFFMANN-LA ROCHE AG, 4070 Basel (CH)
(72) Erfinder: Breu, Volker, 79418 Schliengen (DE); Burri, Kaspar, 4102 Binningen (CH); Cassal, Jean-Marie, 68100 Mulhouse (FR); Clozel, Martine, 68300 St. Louis (FR); Hirth, Georges, 68330 Huningue (FR); Löffler, Bernd-Michael, 79206 Oberrimsingen (DE); Müller, Marcel, 4402 Frenkendorf (CH); Neidhart, Werner, Dr., 68220 Hagenthal le Bas (FR); Ramuz, Henri, 4127 Birsfelden (CH)
(74) Vertreter: Witte, Hubert, Dr.

(56) Entgegenhaltungen:
- EP-A- 0 526 708
- EP-A- 0 601 386
- EP-A- 0 633 259
- EP-A- 0 658 548

## Beschreibung

Die vorliegende Erfindung betrifft neue Sulfonamide und deren Verwendung als Heilmittel. Insbesondere betrifft die Erfindung neue Verbindungen der Formel I 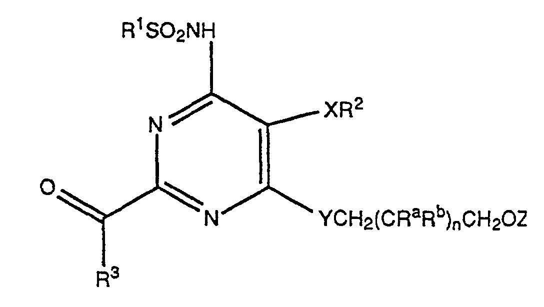 worin
R¹ Phenyl, substituiertes Phenyl oder Heterocyclyl;
R² Phenyl oder substituiertes Phenyl;
R³ Hydroxy, nieder-Alkoxy oder einen Rest -NR⁴R⁵;
R⁴ Wasserstoff oder einen Rest -R⁶;
R⁵ Wasserstoff oder einen Rest -(CH₂)ₘR⁶, oder
R⁴ und R⁵ gemeinsam mit dem damit verbundenen N-Atom einen N-heterocyclischen Rest;
R⁶ Phenyl, substituiertes Phenyl, Cycloalkyl, Heterocyclyl, nieder-Alkyl, Hydroxy-nieder-alkyl, Amino-nieder-alkyl, Carboxy-nieder-alkyl oder nieder-Alkoxycarbonyl-nieder-alkyl;
R^{a} Wasserstoff, nieder-Alkyl oder Hydroxy;
R^{b} Wasserstoff oder nieder-Alkyl;
X Sauerstoff oder Schwefel ;
Y Sauerstoff oder Schwefel;
Z Wasserstoff, nieder-Alkyl, Aryl, Aryl-nieder-alkyl, Heterocyclyl oder Heterocyclyl-nieder-alkyl;
m 0, 1 oder 2; und
n 0, 1 oder 2
bedeuten;
und pharmazeutisch anwendbare Salze davon.

Phenylreste können durch nieder-Alkyl, nieder-Alkoxy, Methylendioxy, Aethylendioxy, nieder-Alkanoyl, Hydroxy, Amino, mono- oder di-nieder-Alkylamino und/oder Halogen substituiert sein. Der hier verwendete Ausdruck "nieder" bezeichnet Gruppen mit 1-7 C-Atomen, vorzugsweise 1-4 C-Atomen. Alkyl- und Alkoxygruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Halogen bezeichnet Fluor, Chlor, Brom und Jod, wobei Chlor bevorzugt ist.

Beispiele von Heterocyclylresten sind mono- oder bicyclische 5- und 6-gliedrige heterocyclische Reste mit Sauerstoff, Stickstoff oder Schwefel als Heteroatom, wie 2- und 3-Furyl, Pyrimidinyl, 2-, 3- und 4-Pyridyl, 2-Tetrazolyl-4-pyridyl, 1,2- und 1,4-Diazinyl, Morpholino, 2- und 3-Thienyl, Isoxazolyl, Oxazolyl, Thiazolyl, Imidazolyl, Pyrrolyl und Tetrazolyl, die z.B. durch nieder-Alkyl, nieder-Alkanoyl, Hydroxy, nieder-Alkanoyloxy, nieder-Alkoxy, nieder-Alkoxycarbonyl, Formyl, Amino, mono- oder di-nieder-Alkylamino oder Halogen substituiert sein können. Mit R⁴ und R⁵ gebildete N-heterocyclische Reste sind vorzugsweise monocylische 6-gliedrige Heterocyclylreste, die ein weiteres Sauerstoff- oder Stickstoffatom enthalten können, wie Morpholino, Piperidino, Piperazino und N⁴-nieder-Alkylpiperazino.

Bevorzugte Reste R¹ sind durch nieder-Alkyl oder nieder-Alkoxy substituiertes Phenyl und monocyclische, ein Stickstoffatom enthaltende Heterocyclreste, wie Pyridyl, insbesondere 2-Pyridyl, die durch nieder-Alkyl, vorzugsweise einfach, substituiert sein können. Bevorzugte Reste R² sind durch nieder-Alkoxy und /oder Halogen substituiertes Phenyl. Bevorzugte Reste R³ sind Hydroxy und -NR⁴R⁵, wobei R⁴ Wasserstoff und R⁵ Phenyl oder Tetrazolyl ist. R^{a} ist vorzugsweise Wasserstoff oder Hydroxy. R^{b} ist vorzugsweise Wasserstoff. X und Y sind vorzugsweise Sauerstoff. Z ist vorzugsweise Wasserstoff. n ist vorzugsweise 0 oder 1.

Die Verbindungen der Formel I und ihre Salze sind Hemmstoffe für Endothelin-Rezeptoren. Sie können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

Die Verbindungen der Formel I und ihre Salze können erfindungsgemäss dadurch hergestellt werden, dass man in einer Verbindung der Formel II 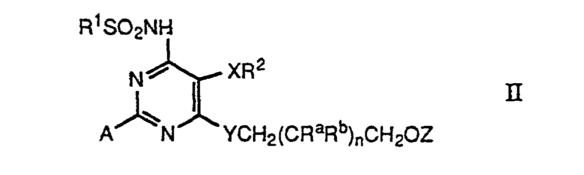 worin A einen in eine Carboxylgruppe überführbaren Rest darstellt, und R¹, R², X, Y, Z, R^{a}, R^{b} und n die oben angegebene Bedeutung haben,
den Rest A in eine Carboxylgruppe umwandelt, gegebenenfalls in der so erhaltenen Verbindung der Formel I, in der R³ Hydroxy ist, die Hydroxygruppe R³ in eine nieder-Alkoxygruppe oder eine Gruppe -NR⁴R⁵ oder in ein pharmazeutisch anwendbares Salz umwandelt.

Beispiele für Reste A sind insbesondere die Formylgruppe und der Rest Furan-2-yl. Diese Reste können in an sich bekannter Weise durch Behandlung mit Oxidationsmitteln in eine Carboxygruppe umgewandelt werden, wobei im Ausgangsmaterial der Formel II anwesende Gruppen, die gegen das angewandte Oxidationsmittel nicht inert sind. wie Hydroxygruppen, zweckmässig geschützt werden. Ein geeignetes Oxidationsmittel für die Oxidation einer Formylgruppe zu einer Carboxylgruppe ist z.B. Kaliumpermanganat. Die Oxidation kann in einem geeigneten inerten Lösungsmittel, wie Benzol in Gegenwart eines Kronenäthers, bei Raumtemperatur durchgeführt werden. Die in der Formel I explizit angegebene Hydroxygruppe sowie als R^{a} und/oder in R¹ anwesende Hydroxygruppen können in an sich bekannter Weise in Form eines Esters, wie des Acetats oder als Ketal, z.B. als Acetonid, geschützt werden. Eine Furan-2-yl-Gruppe kann durch Behandlung mit Perjodat, z.B. Na-Perjodat in Gegenwart von Rutheniumtrichlorid in einem Zweiphasensystem, enthaltend CCl₄, Acetonitril und Wasser zur Carboxylgrupe oxidiert werden.

Die Verbindungen der Formel II sind neu und ebenfalls Gegenstand der Erfindung. Verbindungen der Formel II, in denen A ein Formylrest ist, können aus entsprechenden Methyl-Verbindungen (vgl. die europäische Patentpublikation EP-A-0526708) durch Oxidation, z.B. mit Selendioxid, hergestellt werden (vgl. die europäische Patentpublikation EP-A-0601386). Verbindungen der Formel II, in denen A der Furan-2-yl-Rest und Y Sauerstoff oder Schwefel ist, können ausgehend von Verbindungen der Formel

CH₃COOCH(R²X)COOCH₃

durch Kondensation mit 2-Furanylamidin zu einem 2-Furan-2-yl-(5-XR²)-pyrimidin-4,6-diol, Austausch der OH-Gruppen gegen Cl durch Behandlung mit POCl₃, Umsetzung des 4,6-Dichlorpyrimidinderivats mit einem Sulfonamidsalz der Formel R¹SO₂NHM und danach mit einer Verbindung der Formel

MY'CH₂(CR^{a}R^{b})ₙCH₂OB

worin Y' Sauerstoff oder Schwefel, M ein Kation, z.B. ein Alkalikation wie Na⁺ oder K⁺, und B eine Schutzgruppe darstellen und wobei eine gegebenenfalls durch R^{a} dargestellte Hydroxygruppe in geschützter Form vorliegt,
hergestellt werden.

Die so gebildete Carboxygruppe kann in an sich bekannter Weise in einen nieder-Alkylester (R³ = nieder Alkoxy) oder ein Amid (R³ = -NR⁴R⁵) oder in ein pharmazeutisch anwendbares Salz umgewandelt werden. Beispiele von pharmazeutisch anwendbaren Salzen sind Alkalisalze, wie das Na- und das K-Salz, und Erdalkalisalze, wie das Ca- und das Mg-Salz.

Die Verbindungen der Formel I zeigen eine selektive Hemmwirkung auf die Endothelinrezeptoren A und B (ET_{A} und ET_{B}) was mit den nachstehend beschriebenen Versuchsanordnungen gezeigt werden kann:

### I: Hemmung der Endothelin-Bindung am recombinanten ET_{A}-Rezeptor

Eine cDNA, die für humanen ETA-Rezeptor von menschlicher Plazenta kodiert, wurde kloniert (M. Adachi, Y.-Y. Yang, Y. Furuichi und C-Miyamoto, BBRC 180, 1265-1272) und im Baculovirus-Insektenzellen-System exprimiert. Baculovirus-infizierte Insektenzellen aus einem 23 l Fermenter werden 60 Stunden nach der Infektion abzentrifugiert (3 000 x g, 15 Minuten, 4°C), in Tris-Puffer (5 mM, pH 7.4, 1 mM MgCl₂) resuspendiert und erneut zentrifugiert. Nach erneuter Resuspension und Zentrifugation werden die Zellen in 800 ml des gleichen Puffers suspendiert und bei -120°C eingefroren. Der Aufbruch der Zellen erfolgt beim Auftauen der Suspension in diesem hypotonen Puffergemisch. Nach wiederholtem Gefrier/Auftau-Zyklus wird die Suspension homogenisiert und zentrifugiert (25 000 x g, 15 Minuten, 4°C). Nach Suspension in Tris-Puffer (75 mM, pH 7.4, 25mM MgCl₂, 250 mM Saccharose) werden 1 ml Aliquots (Proteingehalt ca. 3.5 mg/ml) bei -85°C aufbewahrt.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM MnCl₂, 1 mM EDTA und 0,5% Rinderserumalbumin) resuspendiert. 100 µl dieser Membransuspension, enthaltend 5 µg Protein werden mit 50 µl ¹²⁵I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

### II. Hemmung der Endothelin-Bindung an Human-Placentamembranen (ET_{B}-Rezeptor) (vgl. Life Sci 44:1429 (1989))

Humanplacenta wird in 5 mM Tris-Puffer, pH 7.4, der 1 mM MgCl₂ und 250 mM Sucrose enthält, homogenisiert. Das Homogenisat wird mit 3000 g 15 Minuten bei 4°C zentrifugiert, der die Plasmamembranfraktion enthaltende Ueberstand mit 72000 g 30 Minuten zentrifugiert und der Bodenkörper mit 75 mM Tris-Puffer, pH 7.4, der 25 mM MgCl₂ enthält, gewaschen. Danach wird der aus jeweils 10 g Originalgewebe erhaltene Bodenkörper in 1 ml 75 mM Tris-Puffer pH 7.4, enthaltend 25 mM MgCl₂ und 250 mM Sucrose, suspendiert und in 1-ml-Aliquots bei -20°C eingefroren.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM MnCl₂, 1 mM EDTA und 0,5% Rinderserumalbumin) resuspendiert. 100 µl dieser Membransuspension, enthaltend 35 µg Protein werden mit 50 µl ¹²⁵I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

In der Tabelle 1 ist die in diesen Versuchsanordnungen ermittelte Hemmwirkung am ET_{A} und ET_{B} - Rezeptor von Verbindungen der Formel I als IC₅₀ angegeben, d.h., als Konzentration [µM] die erforderlich ist, 50% der spezifischen Bindung von ¹²⁵I-Endothelin zu hemmen.

**Tabelle 1**

| Verbindung von Beispiel | ET_{A} IC₅₀ [µM] | ET_{B} IC₅₀ [µM] |
|---|---|---|
| 1 | 73 | 0,08 |
| 2 | 1,83 | 0,062 |
| 5 | 16,4 | 0,29 |
| 6 | 38 | 0,63 |
| 7 | 18,2 | 0,3 |
| 8 | 13 | 0,06 |
| 9 | 21,4 | 0,086 |
| 10 | 13 | 0,14 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, insbesondere pulmonärer Hochdruck, sowie subarachnoidale Hämorrhagie. Weitere Indikationen zur Anwendung der erfindungsgemässen Verbindungen sind Koronarerkrankungen, Herzinsuffizienz, renale und myocardiale Ischämie, Niereninsuffizienz, cerebrale Ischämie, Hirninfarkt, Migräne und Raynaud-Syndrom. Die erfindungsgemässen Verbindungen können auch bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei Dialyse und bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele erläutern die Erfindung weiter.

### Beispiel 1

a) Zu einer Lösung von 4,6 g Natrium in 150 ml Methanol wurden 25 g Resorcinmonomethyläther und 37 g Chlormalonsäuredimethylester gegeben. Das Reaktionsgemisch wurde bei 45°C unter Argon 4 Stunden gerührt, filtriert, und zwischen Toluol-Wasser verteilt. Die organische Phase wurde mit Natriumsulfat getrocknet, und das Lösungsmittel abdestilliert. Man erhielt 40,7 g Dimethyl-2-(3-Methoxy-phenoxy)-malonat als Oel, MS: (M+H)⁺ 255.
b) Zu einer Natriummethylatlösung aus 450 ml Methanol und 11,3 g Natrium wurden 17 g Acetamidinhydrochlorid und 40 g Dimethyl-2-(3-Methoxy-phenoxy)-malonat gegeben. Das Reaktionsgemisch wurde 2 Stunden bei 20°C gerührt, eingeengt und zwischen Toluol-Wasser verteilt. Die wässrige Phase wurde auf pH 4 mit 2N HCl gestellt, und bei 0°C 16 Stunden gerührt. Der Niederschlag wurde abfiltriert, mit Wasser und Acther gewaschen und getrocknet. Man erhielt 25,7 g 6-Hydroxy-5-(3-methoxy-phenoxy)-2-methyl-3H-pyrimidin-4-on als weissen Festoff, MS: (M+H)⁺ 249.
c) Zu einer Lösung von 11,9 g 6-Hydroxy-5-(3-methoxy-phenoxy)-2-methyl-3H-pyrimidin-4-on in 150 ml Dioxan wurden 24,5 ml Hünig-Base und 23,6 ml POCl₃ gegeben. Das Reaktionsgemisch wurde 16 Stunden bei 120°C gerührt, danach das überschüssige Reagens und das Dioxan abdestilliert. Der Rückstand wurde zweimal mit Toluol eingeengt, und zwischen Chloroform-Wasser verteilt. Die organische Phase wurde mit NaHCO₃ und mit Wasser gewaschen, getrocknet und eingeengt. Der Rückstand wurde über Kieselgel mit Hexan und Dichlormethan gereinigt. Man erhielt 9,1 g 4,6-Dichlor-5-(3-methoxy-phenoxy)-2-methyl-pyrimidin als gelbliches Oel, MS: (M+H)⁺ 286.
d) 9 g 4,6-Dichlor-5-(3-methoxy-phenoxy)-2-methyl-pyrimidin und 17 g p-tert.-Butylsulfonamid-K in 35 ml trockenem Dimethylsulfoxid wurden unter Argon 3 Stunden auf 120°C erwärmt. Danach wurde das DMSO abdestilliert, der Rückstand zwischen Aethylacetat und 1N Salzsäure verteilt und die organische Phase neutral gewaschen. Die organische Phase wurde getrocknet, das Lösungsmittel abgedampft und der Rückstand mit 35 ml Methanol versetzt. Man erhielt 11,1 g p-tert.Butyl-N-[6-chlor-5-(m-methoxyphenoxy)-2-methyl-4-pyrimidinyl-benzolsulfonamid, Smp. 170°C.
e) Zu einer Natriumglykolatlösung aus 35 g Aethylenglykol und 1,66 g Natrium wurden 11 g p-tert.Butyl-N-[6-chlor-5-(m-methoxyphenoxy)-2-methyl-4-pyrimidinyl]benzolsulfonamid gegeben. Das Reaktionsgemisch wurde unter Argon 16 Stunden bei 95°C gerührt, danach mit 100 ml 1N Salzsäure und 50 ml Wasser versetzt, und mit 2 x 100 ml Aethylacetat extrahiert. Die organische Phase wurde mit Wasser gewaschen, getrocknet und eingedampft. Der Rückstand wurde aus Dichlormethan-Isopropyläther kristallisiert. Man erhielt 8,8 g p-tert.Butyl-N-[6-(2-hydroxyäthoxy)-2-methyl-5-(m-methoxyphenoxy)-4-pyrimidinyl]benzolsulfonamid, Smp. 139°C, MS: (M+H)⁺ 488.
f) Zu einer Lösung von 1 g p-tert.Butyl-N-[6-(2-hydroxyäthoxy)-2-methyl-5-(m-methoxy-phenoxy)-4-pyrimidinyl]benzolsulfonamid in 40 ml Dichlormethan wurden 0,2 g Dimethylaminopyridin und 3,9 ml Acetanhydrid gegeben. Das Reaktionsgemisch wurde 3 Stunden bei 20°C gerührt, und mit einer ges. NaHCO₃-Lösung auf pH 7 gestellt. Die organische Phase wurde mit Wasser gewaschen, getrocknet und eingedampft. Der Rückstand wurde über Kieselgel mit Chloroform gereinigt. Man erhielt 1,1 g Essigsäure-2-[6-acetyl-(4-tert.butyl-phenylsulfonyl)-amino]-5-(3-methoxy-phenoxy)-2-methylpyrimidin-4-yloxy]-äthylester als Schaum, MS: (M+H)⁺ 572.
g) 0,2 g Essigsäure-2-[6-acetyl-(4-tert.butyl-phenylsulfonyl)-amino]-5-(3-methoxy-phenoxy)-2-methyl-pyrimidin-4-yloxy]-äthylester und 0,6 g Selendioxid in 10 ml Dioxan wurden in einem Autoklaven bei 140°C während 50 Stunden gerührt. Das Reaktionsgemisch wurde filtriert und das Filtrat eingeengt. Der Rückstand wurde zwischen Aethylacetat und Wasser verteilt. Die organische Phase wurde getrocknet, das Lösungsmittel abgedampft und der Rückstand über Kieselgel mit Chloroform-Methanol 95:5 gereinigt. Man erhielt 0,27 g Essigsäure-2-[6-(4-tert.butyl-phenylsulfonylamino)-2-formyl-5-(3-methoxy-phenoxy)-pyrimidin-4-yloxy]-äthylester als Schaum, MS: (M+H)⁺ 544.
h) 0,27 g Essigsäure-2-[6-(4-tert.butyl-phenylsulfonylamino)-2-formyl-5-(3-methoxy-phenoxy)-pyrimidin-4-yloxy]-äthylester und 0,19 g Dicyclohexyl-18-crown-6 in 15 ml Benzol wurden mit 0,078 g Kaliumpermanganat 16 Stunden bei 20°C gerührt. Das Reaktionsgemisch wurde zwischen Toluol und Wasser verteilt. Die organische Phase wurde getrocknet, abgedampft und der Rückstand über Kieselgel mit Chloroform-Methanol gereinigt. Man erhielt 0,09 g 4-(2-Acetoxy-äthoxy)-6-(4-tert.butyl-phenylsulfonylamino)-5-(3-methoxy-phenoxy)-pyrimidin-2-carbonsäure als Schaum, MS: (M-H)⁻ 558.
i) 0,09 g 4-(2-Acetoxy-äthoxy)-6-(4-tert.butyl-phenylsulfonylamino)-5-(3-methoxy-phenoxy)-pyrimidin-2-carbonsäure wurden in 4 ml Methanol und 1,5 ml Wasser mit 0,05 g Natriumcarbonat während 2 Stunden bei 20°C gerührt. Das Methanol wurde abgedampft und der Rückstand zwischen Chloroform und wässriger 1N Salzsäure verteilt. Die organische Phase wurde getrocknet und eingedampft. Der Rückstand wurde über Kieselgel mit Chloroform-Methanol-Wasser, 60:35:5 gereinigt. Man erhielt 0,034 g 6-(4-tart.Butyl-phenylsulfonylamino)-4-(2-hydroxy-äthoxy)-5-(3-methoxyphenoxy)-pyrimidin-2-carbonsäure, MS: (M-H)- 515,9.

### Beispiel 2

In Analogie zu Beispiel 1 wurde die 4-(4-tert.Butyl-phenylsulfonylamino)-5-(2-chlor-5-methoxy-phenoxy)-6-(2-hydroxy-äthoxy)-pyrimidin-2-carbonsäure, MS: (M-H)- 550,2 aus 2-Chlor-5-methoxy-phenol über folgende Zwischenstufen hergestellt:
a) Dimethyl-(2-Chlor-5-methoxy-phenoxy)-malonat,
b) 6-Hydroxy-5-(2-chlor-5-methoxy-phenoxy)-2-methyl-3H-pyrimidin-4-on,
c) 4,6-Dichlor-5-(2-chlor-5-methoxy-phenoxy)-2-methyl-pyrimidin,
d) p-tert.Butyl-N-[6-chlor-5-(2-chlor-5-methoxyphenoxy)-2-methyl-4-pyrimidinyl]benzolsulfonamid,
e) p-tert.Butyl-N-[6-(2-hydroxyäthoxy)-2-methyl-5-(2-chlor-5-methoxyphenoxy)-4-pyrimidinyl]benzolsulfonamid,
f) Essigsäure-2-[6-(4-tert.butyl-phenylsulfonylamino)-5-(2-chlor-5-methoxy-phenoxy)-2-methyl-pyrimidin-4-yloxy]-äthylester,
g) Essigsäure-2-[6-(4-tert.butyl-phenylsulfonylamino)-5-(2-chlor-5-methoxy-phenoxy)-2-formyl-pyrimidin-4-yloxy]-äthylester,
h) 4-(2-Acetoxy-äthoxy)-6-(4-tert.butyl-phenylsulfonylamino)-5-(2-chlor-5-methoxy-phenoxy)-pyrimidin-2-carbonsäure.

### Beispiel 3

In Analogie zu Beispiel 1 wurde die 4-(2-Hydroxy-äthoxy)-5-(3-methoxyphenoxy)-6-(4-methoxy-phenylsulfonylamino)-pyrimidin-2-carbonsäure, MS: (M-H)⁻ 490,3 aus 4,6-Dichlor-5-(3-methoxy-phenoxy)-2-methyl-pyrimidin über folgende Zwischenstufen hergestellt:
a) N-[6-Chlor-5-(3-methoxy-phenoxy)-2-methyl-pyrimidin-4-yl]-4-methoxybenzolsulfonamid,
b) N-[6-(2-Hydroxy-äthoxy)-5-(3-methoxy-phenoxy)-2-methyl-pyrimidin-4-yl]-4-methoxy-benzolsulfonamid,
c) Essigsäure-2-[6-(4-methoxy-phenylsulfonylamino)-5-(m-methoxyphenoxy)-2-methyl-pyrimidin-4-yloxy]-äthylester,
d) Essigsäure-2-[6-(4-methoxy-phenylsulfonylamino)-5-(m-methoxyphenoxy)-2-formyl-pyrimidin-4-yloxy]-äthylester,
e) 4-(2-Acetoxy-äthoxy)-6-(4-methoxy-phenylsulfonylamino)-5-(m-methoxyphenoxy)-pyrimidin-2-carbonsäure.

### Beispiel 4

a) In Analogie zu Beispiel 1b) wurde aus 34 g Dimethyl-2-(3-Methoxyphenoxy)-malonat und 23 g 2-Furanylamidin, 15,6 g 2-Furan-2-yl-5-(3-methoxy-phenoxy)-pyrimidin-4,6-diol als Schaum, MS: (M+H)⁺ 300, erhalten.
b) In Analogie zu Beispiel 1c) wurde aus 15,5 g 2-Furan-2-yl-5-(3-methoxyphenoxy)-pyrimidin-4,6-diol und 43 ml POCl₃, 15,9 g 4,6-Dichlor-2-furan-2-yl-5-(3-methoxy-phenoxy)-pyrimidin, Smp. 106°C, MS: (M+H)⁺ 338, erhalten.
c) In Analogie zu Beispiel 1d) wurde aus 1 g 4,6-Dichlor-2-furan-2-yl-5-(3-methoxy-phenoxy)-pyrimidin und 1,5 g p-tert.Butylsulfonamid-K, 1,34 g 4-tert.Butyl-N-[6-chlor-2-furan-2-yl-5-(3-methoxy-phenoxy)-pyrimidin-4-yl]-benzolsulfonamid, MS: (M-H)⁻ 513, erhalten.
d) In Analogie zu Beispiel 1e) wurde aus 0,3 g 4-tert.Butyl-N-[6-chlor-2-furan-2-yl-5-(3-methoxy-phenoxy)-pyrimidin-4-yl]-benzolsulfonamid und 12 ml DL-Isopropyliden-glycerol, 0,25 g (RS)-4-tert.Butyl-N-[6-(2,2-dimethyl-[1,3]dioxolan-4-ylmethoxy)-2-furan-2-yl-5-(3-methoxy-phenoxy)-pyrimidin-4-yl]-benzolsulfonamid, Smp. 135°C, MS: (M-H)⁻ 608, erhalten.
e) Zu einer Lösung von 0,25 g (RS)-4-tert.Butyl-N-[6-(2,2-dimethyl-[1,3]-dioxolan-4-ylmethoxy)-2-furan-2-yl-5-(3-methoxy-phenoxy)-pyrimidin-4-yl]-benzolsulfonamid in 3,6 ml CCl₄ und 3,6 ml Acetonitril wurde eine Lösung von 0,7 g Natriumperjodat und 0,015 g Rutheniumchlorid in 10 ml Wasser gegeben. Das Reaktionsgemisch wurde 1 Stunde bei 20°C gerührt, und mit Methylenchlorid extrahiert. Die organische Phase wurde getrocknet und abgedampft. Der Rückstand wurde über Kieselgel mit Chloroform gereinigt. Man erhielt 0,04 g (RS)-4-(4-tert.Butyl-phenylsulfonylamino-6-(2,2-dimethyl-[1,3]dioxolan-4-ylmethoxy)-5-(3-methoxy-phenoxy)-pyrimidin-2-carbonsäure, MS: (M-H)⁻ 586.
f) Eine Lösung von 0,033 g (RS)-4-(4-tert.Butyl-phenylsulfonylamino-6-(2,2-dimethyl-[1,3]dioxolan-4-ylmethoxy)-5-(3-methoxy-phenoxy)-pyrimidin-2-carbonsäure in 2 ml Dioxan wurde mit 1,3 ml 1N HCl versetzt und 30 Minuten auf 90°C erwärmt. Nach Eindampfen wurde der Rückstand über Kieselgel mit Chloroform-Methanol-Wasser 60:35:5 als Fliessmittel chromatographiert und lieferte 0,007 g (RS)-6-(4-tert.Butyl-phenylsulfonylamino)-4-(2,3-dihydroxy-propoxy)-5-(3-methoxy-phenoxy)-pyrimidin-2-carbonsäure als Schaum, MS: (M-H)⁻ 546.

### Beispiel 5

In Analogie zu Beispiel 4e) wurde aus Essigsäure-2-[6-(4-tert.butylphenylsulfonylamino)-2-furan-2-yl-5-(2-methoxy-phenoxy)-pyrimidin-4-yloxy]-äthylester die 4-(2-Acetoxy-äthoxy)-6-(4-tert.butyl-phenylsulfonylamino)-5-(2-methoxy-phenoxy)-pyrimidin-2-carbonsäure, MS: (M-H)- 558 und daraus in Analogie zu Beispiel 1i) die 6-(4-tert.Butyl-phenylsulfonylamino)-4-(2-hydroxy-äthoxy)-5-(2-methoxy-phenoxy)-pyrimidin-2-carbonsäure, MS: (M-H)⁻ 516,4, erhalten.

### Beispiel 6

Zu einer Lösung von 0,053 g 6-(4-tert.Butyl-phenylsulfonylamino)-4-(2-hydroxy-äthoxy)-5-(3-methoxy-phenoxy)-pyrimidin-2-carbonsäure in 5 ml Acetonitril, wurden 0,02 ml Hünig-Base, 0,052 g Bis-(2-oxo-3-oxazolidinyl)-phosphinsäurechlorid und 0,01 ml Anilin gegeben. Nach 3 Stunden wurde das Reaktionsgemisch abgedampft und zwischen Essigester und Wasser verteilt. Die organische Phase wurde getrocknet, abgedampft, und der Rückstand über Kieselgel mit Chloroform gereinigt. Man erhielt 0,032 g 6-(4-tert.Butyl-phenylsulfonylamino)-4-(2-hydroxy-äthoxy)-5-(3-methoxyphenoxy)-pyrimidin-2-carbonsäure-phenylamid, Smp. 143°C, MS: (M-H)⁻ 591.

### Beispiel 7

In Analogie zu Beispiel 6 wurde das 4-(2-Hydroxy-äthoxy)-5-(3-methoxyphenoxy)-6-(4-methoxy-phenylsulfonylamino)-pyrimidin-2-carbonsäurephenylamid, MS: (M+H)⁺ 567, erhalten.

### Beispiel 8

In Analogie zu Beispiel 6 wurde das 6-(4-tert.Butyl-phenylsulfonylamino)-5-(2-methoxy-phenoxy)-4-(2-hydroxy-äthoxy)-pyrimidin-2-carbonsäure-phenylamid, MS: (M-H)⁻ 591, erhalten.

### Beispiel 9

In Analogie zu Beispiel 6 wurde das 4-(4-tert.Butyl-phenylsulfonylamino)-5-(2-chlor-5-methoxy-phenoxy)-6-(2-hydroxy-äthoxy)-pyrimidin-2-carbonsäure-phenylamid, MS: (M+H)⁺ 628, erhalten.

### Beispiel 10

In Analogie zu Beispiel 6 wurde das 4-(4-tert.Butyl-phenylsulfonylamino)-6-(2-hydroxy-äthoxy)-5-(3-methoxy-phenoxy)-pyrimidin-2-carbonsäure-(1H-tetrazol-5-yl)-amid, MS: (M-H)⁻ 583, erhalten.

### Beispiel 11

In Analogie zu Beispiel 4, Abschnitt c), d), e) wurde aus 4,6-Dichlor-2-furan-2-yl-5-(3-methoxy-phenoxy)-pyrimidin:
a) 5-Isopropyl-pyridin-2-sulfonsäure-[6-chlor-2-furan-2-yl-5-(3-methoxyphenoxy)-pyrimidin-4-yl]-amid
b) 5-Isopropyl-pyridin-2-sulfonsäure-[2-füran-2-yl-6-(2-hydroxy-äthoxy)-5-(3-methoxy-phenoxy)-pyrimidin-4-yl]-amid
c) Essigsäure-2-[6-[acetyl-(5-isopropyl-pyridin-2-ylsulfonyl)-amino]-2-furan-2-yl-5-(3-methoxy-phenoxy)-pyrimidin-4-yloxy]-äthylester
d) 4-(2-Acetoxy-äthoxy)-6-(5-isopropyl-pyridin-2-ylsulfonyl)-amino]-5-(3-methoxy-phenoxy)-pyrimidin-2-carbonsäure
und daraus in Analogie zu Beispiel 1 die 4-(2-Hydroxy-äthoxy)-6-(5-isopropylpyridin-2-ylsulfonylamino)-5-(3-methoxy-phenoxy)-pyrimidin-2-carbonsäure, MS: (M-H)⁻ 503, erhalten.

### Beispiel 12

In Analogie zu Beispiel 4, Abschnitt c), d), e) wurde aus 4,6-Dichlor-2-furan-2-yl-5-(3-methoxy-phenoxy)-pyrimidin:
a) 5-Methyl-pyridin-2-sulfonsäure-[6-chlor-2-furan-2-yl-5-(3-methoxyphenoxy)-pyrimidin-4-yl]-amid
b) 5-Methyl-pyridin-2-sulfonsäure-[2-furan-2-yl-6-(2-hydroxy-äthoxy)-5-(3-methoxy-phenoxy)-pyrimidin-4-yl]-amid
c) Essigsäure-2-[2-furan-2-yl-5-(3-methoxy-phenoxy)-6-(5-methyl-pyridin-2-ylsulfonylamino )-pyrimidin-4-yloxy]-äthylester
d) 4-(2-Acetoxy-äthoxy)-6-(5-methyl-pyridin-2-ylsulfonyl)-amino]-5-(3-methoxy-phenoxy)-pyrimidin-2-carbonsäure
und daraus in Analogie zu Beispiel 1 die 4-(2-Hydroxy-äthoxy)-6-(5-isopropylpyridin-2-ylsulfonylamino)-5-(3-methoxy-phenoxy)-pyrimidin-2-carbonsäure, MS: (M-H)⁻ 475 erhalten.

### Beispiel 13

In Analogie zu Beispiel 6 kann das 4-(2-Hydroxyäthoxy)-5-(3-methoxyphenoxy)-6-(5-methyl-pyridin-2-ylsulfonylamino)pyrimidin-2-carbonsäure (1H-tetrazol-5-yl)amid erhalten werden.

### Beispiel 14

In Analogie zu Beispiel 4 kann die (R,S)-6-(2,3-Dihydroxy-propoxy)-5-(3-methoxyphenoxy)-4-(5-methylpyridin-2-yl-sulfonylamino)-pyrimidin-2-carbonsäure erhalten werden.

### Beispiel 15

In Analogie zu Beispiel 6 kann das 6-(4-tert.Butylphenylsulfonylamino)-4-(2,3-dihydroxypropoxy)-5-(3-methoxyphenoxy)pyrimidin-2-carbonsäure (1H-tetrazol-5-yl)amid erhalten werden.

### Beispiel 16

In Analogie zu Beispiel 6 wurde das 4-tert-Butyl-N-[6-(2-hydroxy-äthoxy)-5-(3-methoxy-phenoxy)-2-(morpholin-4-ylcarboxyl)-pyrimidin-4-yl]-benzolsulfonamid erhalten. MS: (M-H)⁻ 585.

### Beispiel 17

In Analogie zu Beispiel 6 wurde das 4-(4-tert-Butyl-phenylsulfonylamino)-6-(2-hydroxy-äthoxy)-5-(3-methoxy-phenoxy)-pyrimidin-2-carbonsäure-pyridin-3-ylamid erhalten. MS: (M-H)⁻ 592.

### Beispiel 18

In Analogie zu Beispiel 6 wurde das 4-(4-tert-Butyl-phenylsulfonylamino)-6-(2-hydroxy-äthoxy)-5-(3-methoxy-phenoxy)-pyrimidin-2-carbonsäure (3-hydroxy-phenyl)-amid erhalten. MS: (M-H)⁻ 607.

### Beispiel 19

In Analogie zu Beispiel 6 wurde das 4-(4-tert-Butyl-phenylsulfonylamino)-6-(2-hydroxy-äthoxy)-5-(3-methoxy-phenoxy)-pyrimidin-2-carbonsäure (2-hydroxymethyl-phenyl)-amid erhalten. MS: (M+H)⁺ 623.

### Beispiel 20

In Analogie zu Beispiel 6 wurde das 4-(4-tert-Butyl-phenylsulfonylamino)-6-(2-hydroxyäthoxy)-5-(3-methoxy-phenoxy)-pyrimidin-2-carbonsäure (3-hydroxymethyl-phenyl)-amid erhalten. MS (M-H)⁻ 621.

### Beispiel 21

In Analogie zu Beispiel 6 wurde das (RS)-4-(4-tert-Butyl-phenylsulfonylamino)-6-(2,3-dihydroxy-propoxy)-5-(3-methoxy-phenoxy)-pyrimidin-2-carbonsäure-pyridin-3-ylamid erhalten. MS: (M-H)⁻ 622.

### Beispiel 22

In Analogie zu Beispiel 6 wurde das (RS)-4-(4-tert-Butyl-phenylsulfonylamino)-6-(2,3-dihydroxy-propoxy)-5-(3-methoxy-phenoxy)-pyrimidin-2-carbonsäure (3-hydroxy-phenyl)-amid erhalten. MS: (M-H)⁻ 637.

### Beispiel 23

0,2 g 6-(4-tert-Butyl-phenylsulfonylamino)-4-(2-hydroxy-äthoxy)-5-(3-methoxy-phenoxy)-pyrimidin-2-carbonsäure wurden in 5 ml Dimethylacetamid mit 0,04 g Natriumhydrid und 0,07 g 2-Chlorpyrimidin, 3 Stunden bei 20°C gerührt, und mit einer gesättigten NH₄Cl-Lösung neutralisiert. Das Reaktionsgemisch wurde mit Aethylacetat gewaschen. Die wässrige Phase wurde mit HCI 1N auf pH 2 gestellt, und mit Chloroform extrahiert. Die organische Phase wurde getrocknet, das Lösungsmittel abgedampft und der Rückstand über Kieselgel mit Chloroform-Methanol-Wasser, 60:35:5 gereinigt. Man erhielt 0,16 g 4-(4-tert-Butyl-phenylsulfonylamino)-5-(3-methoxy-phenoxy)-6-(2-pyrimidin-2-yloxy-äthoxy)-pyrimidin-2-carbonsäure, MS: (M+H)⁺ 596.

### Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk | 4,0 mg |
| Magnesiumstearat | 1,0 mg |

### Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |

### Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

### Beispiel D

In 3,5 ml Myglyol 812 und 0,08 g Benzylalkohol werden 500 mg Verbindung der Formel I suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

## Patentansprüche

1. Verbindungen der Formel I 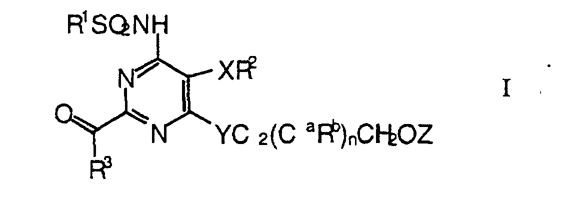 worin
R¹ Phenyl, durch nieder-Alkyl, nieder-Alkoxy, Methylendioxy, Aethylendioxy, nieder-Alkanoyl, Hydroxy, Amino, mono- oder di-nieder-Alkylamino und/oder Halogen substituiertes Phenyl oder Heterocyclyl;
R² Phenyl oder durch nieder-Alkyl, nieder-Alkoxy, Methylendioxy, Aethylendioxy, nieder-Alkanoyl, Hydroxy, Amino, mono- oder di-nieder-Alkylamino und/oder Halogen substituiertes Phenyl;
R³ Hydroxy, nieder-Alkoxy oder einen Rest -NR⁴R⁵;
R⁴ Wasserstoff oder einen Rest -R⁶;
R⁵ Wasserstoff oder einen Rest -(CH₂)ₘR⁶, oder
R⁴ und R⁵ gemeinsam mit dem damit verbundenen N-Atom einen monocylischen 6-gliedrigen Heterocyclylrest, der ein weiteres Sauerstoff- oder Stickstoffatom enthalten und am letzteren Stickstoffatom durch nieder-Alkyl substituiert sein kann;
R⁶ Phenyl, durch nieder-Alkyl, nieder-Alkoxy, Methylendioxy, Aethylendioxy, nieder-Alkanoyl, Hydroxy, Amino, mono- oder di-nieder-Alkylamino und/oder Halogen substituiertes Phenyl, C3 - C8 Cycloalkyl, Heterocyclyl, nieder-Alkyl, Hydroxy-nieder-alkyl, Amino-nieder-alkyl, Carboxy-nieder-alkyl oder nieder-Alkoxycarbonyl-nieder-alkyl;
R^{a} Wasserstoff, nieder-Alkyl oder Hydroxy;
R^{b} Wasserstoff oder nieder-Alkyl;
X Sauerstoff oder Schwefel;
Y Sauerstoff oder Schwefel;
Z Wasserstoff, nieder-Alkyl, Aryl, Aryl-nieder-alkyl, Heterocyclyl oder Heterocyclyl-nieder-alkyl;
m 0, 1 oder 2;
n 0, 1 oder 2;
"Heterocyclyl" einen ggf. durch nieder-Alkyl, nieder-Alkanoyl, Hydroxy, nieder-Alkanoyloxy, nieder-Alkoxy, nieder-Alkoxycarbonyl, Formyl, Amino, mono-oder di-nieder-Alkylamino oder Halogen substituierten mono- oder bicyclischen 5- und 6-gliedrigen heterocyclischen Rest mit Sauerstoff, Stickstoff oder Schwefel als Heteroatom;
"Aryl" ggfs. durch nieder-Alkyl, nieder-Alkoxy, Methylendioxy, Aethylendioxy, nieder-Alkanoyl, Hydroxy, Amino, mono- oder di-nieder-Alkylamino und/oder Halogen substituiertes Phenyl;
und "nieder" Gruppen mit 1-7 C-Atomen
bedeuten;
und pharmazeutisch anwendbare Salze davon.

2. Verbindungen gemäss Anspruch 1, in denen X und Y Sauerstoff sind.

3. Verbindungen gemäss Anspruch 1 oder 2, in denen n 0 oder 1 ist.

4. Verbindungen gemäss einem der Ansprüche 1 bis 3, in denen R¹ durch nieder-Alkyl oder nieder-Alkoxy substituiertes Phenyl ersetzt werden kann.

5. Verbindungen gemäss einem der Ansprüche 1 bis 3 , in denen R¹ einen ggfs. durch nieder-Alkyl substituierten monocyclischen, ein Stickstoffatom enthaltenden Heterocyclylrest, darstellt.

6. Verbindungen gemäss einem der Ansprüche 1 bis 5, in denen R² durch nieder-Alkoxy und/oder Halogen substituiertes Phenyl darstellt.

7. Verbindungen gemäss einem der Ansprüche 1 bis 3, worin R³ Hydroxy oder NR⁴R⁵ darstellt.

8. Verbindungen gemäss einem der Ansprüche 1 bis 6, worin R³ NR⁴R⁵, R⁵-(CH₂)ₘR⁶ und R⁶ Phenyl, substituiertes Phenyl, C₃₋₈-Cycloalkyl oder Heterocyclyl ist.

9. Die Verbindungen gemäss Anspruch 4,
6-(4-tert.Butyl-phenylsulfonylamino)-4-(2-hydroxy-äthoxy)-5-(3-methoxyphenoxy)-pyrimidin-2-carbonsäure,
4-(4-tert.Butyl-phenylsulfonylamino)-5-(2-chlor-5-methoxy-phenoxy)-6-(2-hydroxy-äthoxy)-pyrimidin-2-carbonsäure,
4-(2-Hydroxy-äthoxy)-5-(3-methoxy-phenoxy)-6-(4-methoxyphenylsulfonylamino)-pyrimidin-2-carbonsäure,
(RS)-6-(4-tert.Butyl-phenylsulfonylamino)-4-(2,3-dihydroxy-propoxy)-5-(3-methoxy-phenoxy)-pyrimidin-2-carbonsäure,
6-(4-tert.Butyl-phenylsulfonylamino)-4-(2-hydroxy-äthoxy)-5-(2-methoxyphenoxy)-pyrimidin-2-carbonsäure,
6-(4-tert.Butyl-phenylsulfonylamino)-4-(2-hydroxy-äthoxy)-5-(3-methoxyphenoxy)-pyrimidin-2-carbonsäure-phenylamid,
4-(2-Hydroxy-äthoxy)-5-(3-methoxy-phenoxy)-6-(4-methoxyphenylsulfonylamino)-pyrimidin-2-carbonsäure-phenylamid,
6-(4-tert.Butyl-phenylsulfonylamino)-5-(2-methoxy-phenoxy)-4-(2-hydroxy-äthoxy)-pyrimidin-2-carbonsäure-phenylamid,
4-(4-tert.Butyl-phenylsulfonylamino)-5-(2-chlor-5-methoxy-phenoxy)-6-(2-hydroxy-äthoxy)-pyrimidin-2-carbonsäure-phenylamid,
4-(4-tert.Butyl-phenylsulfonylamino)-6-(2-hydroxy-äthoxy)-5-(3-methoxyphenoxy)-pyrimidin-2-carbonsäure-( 1H-tetrazol-5-yl)-amid.

10. Die Verbindungen gemäss Anspruch 5,
4-(2-Hydroxy-äthoxy)-6-(5-isopropyl-pyridin-2-ylsulfonylamino)-5-(3-methoxy-phenoxy)-pyrimidin-2-carbonsäure,
4-(2-Hydroxy-äthoxy)-6-(5-isopropyl-pyridin-2-ylsulfonylamino)-5-(3-methoxy-phenoxy)-pyrimidin-2-carbonsäure.

11. Verbindungen der Formel 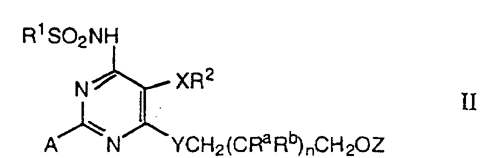 worin A Formyl oder Furan-2-yl und R¹, R², X, Z, R^{a}, R^{b}, Y und n die in Anspruch 1 angegebene Bedeutung haben und wobei eine in der Formel II durch OZ dargestellte Hydroxygruppe und eine gegebenenfalls als R^{a} und/oder in R¹ anwesende Hydroxygruppe als Ester oder Ketal in geschützter Form vorliegen können.

12. Die Verbindungen der Ansprüche 1-10 zur Anwendung als Heilmittel.

13. Pharmazeutische Präparate, enthaltend eine Verbindung der Ansprüche 1-10 und übliche Träger- und Hilfsstoffe.

14. Verwendung von Verbindungen der Ansprüche 1-10 als Wirkstoffe bei der Herstellung von Heilmitteln zur Behandlung von Erkrankungen, die mit Endothelin-Aktivitäten assoziiert sind, insbesondere Kreislauferkrankungen wie Hypertonie, Ischämie, Vasopasmen und Angina pectoris.

15. Verfahren zur Herstellung von Verbindungen der Ansprüche 1-10, **dadurch gekennzeichnet, dass** man
in einer Verbindung der Formel II 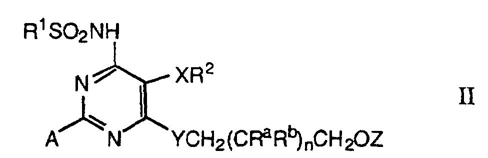
worin A Formyl oder Furan-2-yl darstellt, und R¹, R², X, Y, Z, R^{a}, R^{b} und n die in Anspruch 1 angegebene Bedeutung haben,und wobei eine in der Formel II durch OZ dargestellte Hydroxygruppe und eine gegebenenfalls als R^{a} und/oder in R¹ anwesende Hydroxygruppe als Ester oder Ketal in geschützter Form vorliegen können,den Rest A in eine Carboxylgruppe umwandelt, gegebenenfalls in der so erhaltenen Verbindung der Formel I, in der R³ Hydroxy ist, die Hydroxygruppe R³ in eine nieder-Alkoxygruppe oder eine Gruppe -NR⁴R⁵ umwandelt, notwendigenfalls eine veresterte oder ketalisierte Hydroxygruppe in freies Hydroxy überführt und ggfs. eine Verbindung der Formel I in ein pharmazeutisch anwendbares Salz umwandelt

## Claims

1. Compounds of the formula 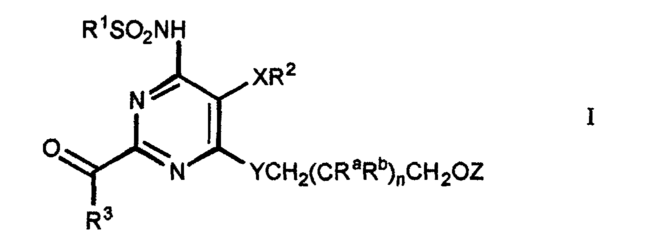 wherein
R¹ signifies phenyl, phenyl substituted by lower-alkyl, lower-alkoxy, methylenedioxy, ethylenedioxy, lower-alkanoyl, hydroxy, amino, mono- or di-lower-alkylamino and/or halogen, or heterocyclyl;
R² signifies phenyl or phenyl substituted by lower-alkyl, lower-alkoxy, methylenedioxy, ethylenedioxy, lower-alkanoyl, hydroxy, amino, mono- or di-lower-alkylamino and/or halogen;
R³ signifies hydroxy, lower-alkoxy or a residue -NR⁴R⁵;
R⁴ signifies hydrogen or a residue -R⁶;
R⁵ signifies hydrogen or a residue -(CH₂)ₘR⁶, or
R⁴ and R⁵ together with the N atom to which they are attached signify a monocyclic 6-membered heterocyclyl residue which can contain a further oxygen or nitrogen atom and which can be substituted on the latter nitrogen atom by lower-alkyl;
R⁶ signifies phenyl, phenyl substituted by lower-alkyl, lower-alkoxy, methylenedioxy, ethylenedioxy, lower-alkanoyl, hydroxy, amino, mono- or di-lower-alkylamino and/or halogen, C3-C8 cycloalkyl, heterocyclyl, lower-alkyl, hydroxy-lower-alkyl, amino-lower-alkyl, carboxy-lower-alkyl or lower-alkoxycarbonyl-lower-alkyl;
R^{a} signifies hydrogen, lower-alkyl or hydroxy;
R^{b} signifies hydrogen or lower-alkyl;
X signifies oxygen or sulphur;
Y signifies oxygen or sulphur;
Z signifies hydrogen, Lower-alkyl, aryl, aryl-lower-alkyl, heterocyclyl or heterocyclyl-lower-alkyl;
m signifies 0, 1 or 2; and
n signifies 0, 1 or 2;
"heterocyclyl" signifies a mono- or bicyclic 5- and 6-membered heterocyclic residue with oxygen, nitrogen or sulphur as the hetero atom optionally substituted by lower-alkyl, lower-alkanoyl, hydroxy, lower-alkanoyloxy, lower-alkoxy, lower-alkoxycarbonyl, formyl, amino, mono- or di-lower-alkylamino or halogen;
"aryl" signifies phenyl optionally substituted by lower-alkyl, lower-alkoxy, methylenedioxy, ethylenedioxy, lower-alkanoyl, hydroxy, amino, mono- or di-lower-alkylamino and/or halogen;
and "lower" signifies groups with 1-7 C atoms;
and pharmaceutically usable salts thereof.

2. Compounds according to claim 1, in which X and Y are oxygen.

3. Compounds according to claim 1 or 2, in which n is 0 or 1.

4. Compounds according to any one of claims 1 to 3, in which R¹ can be replaced by phenyl substituted by lower-alkyl or lower-alkoxy.

5. Compounds according to any one of claims I to 3, in which R¹ represents a monocyclic heterocyclyl residue which contains a nitrogen atom and which is optionally substituted by lower-alkyl.

6. Compounds according to any one or claims 1 to 5, in which R² represents phenyl substituted by lower-alkoxy and/or halogen.

7. Compounds according to any one of claims 1 to 3, wherein R³ signifies hydroxy or NR⁴R⁵.

8. Compounds according to any one of claims 1 to 6, wherein R³ signifies NR⁴R⁵, R⁵ signifies -(CH₂)ₘR⁶ and R⁶ signifies phenyl, substituted phenyl, C₃₋₈-cycloalkyl or heterocyclyl.

9. The compounds according to claim 4,
6-(4-tert.butyl-phenylsulphonylamino)-4-(2-hydroxy-ethoxy)-5-(3-methoxyphenoxy)-pyrimidine-2-carboxylic acid,
4-(4-tert.butyl-phenylsulphonylamino)-5-(2-chloro-5-methoxy-phenoxy)-6-(2-hydroxy-ethoxy)-pyrimidine-2-carboxylic acid,
4-(2-hydroxy-ethoxy)-5-(3-methoxy-phenoxy)-6-(4-methoxy-phenylsulphonylamino)-pyrimidine-2-carboxylic acid,
(RS)-6-(4-tert.butyl-phenylsulphonylamino)-4-(2,3-dihydroxy-propoxy)-5-(3-methoxy-phenoxy)-pyrimidine-2-carboxylic acid,
6-(4-tert.butyl-phenylsulphonylamino)-4-(2-hydroxy-ethoxy)-5-(2-methoxyphenoxy)-pyrimidine-2-carboxylic acid,
6-(4-tert.butyl-phenylsulphonylamino)-4-(2-hydroxy-ethoxy)-5-(3-methoxyphenoxy)-pyrimidine-2-carboxylic acid phenylamide,
4-(2-hydroxy-ethoxy)-5-(3-methoxy-phenoxy)-6-(4-methoxy-phenylsulphonylamino)-pyrimidine-2-carboxylic acid phenylamide,
6-(4-tert.butyl-phenylsulphonylamino)-5-(2-methoxy-phenoxy)-4-(2-hydroxyethoxy)-pyrimidine-2-carboxylic acid phenylamide,
4-(4-tert.butyl-phenylsulphonylamino)-5-(2-chloro-5-methoxy-phenoxy)-6-(2-hydroxy-ethoxy)-pyrimidine-2-carboxylic acid phenylamide,
4-(4-tert.butyl-phenylsulphonylamino)-6-(2-hydroxy-ethoxy)-5-(3-methoxyphenoxy)-pyrimidine-2-carboxylic acid (1H-tetrazol-5-yl)-amide.

10. The compounds according to claim 5,
4-(2-hydroxy-ethoxy)-6-(5-isopropyl-pyridin-2-ylsulphonylamino)-5-(3-methoxyphenoxy)-pyrimidine-2-carboxylic acid,
4-(2-hydroxy-ethoxy)-6-(5-isopropyl-pyridin-2-ylsulphonylamino)-5-(3-methoxyphenoxy)-pyrimidine-2-carboxylic acid.

11. Compounds of the formula 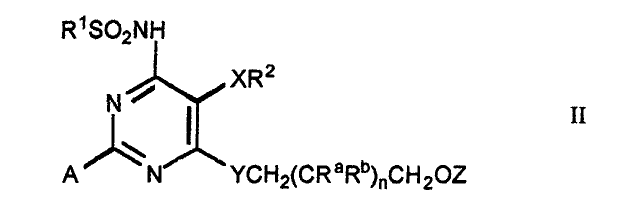
wherein A [represents] formyl or furan-2-yl and R¹, R², X, Z, R^{a}, R^{b}, Y and n have the significance given in claim 1 and wherein a hydroxy group represented in formula II by OZ and a hydroxy group which may be present as R^{a} and/or in R¹ can be present in protected form as an ester or ketal.

12. The compounds of claims 1-10 for use as medicaments.

13. A pharmaceutical preparation containing a compound of claims 1-10 and usual carrier materials and adjuvants.

14. The use of compounds of claims 1-10 as active ingredients for the production of medicaments for the treatment of disorders which are associated with endothelin activities, especially circulatory disorders such as hypertension, ischemia, vasospasms and angina pectoris.

15. A process for the manufacture of compounds of claims 1-10, **characterized by** converting the residue A in a compound of formula II 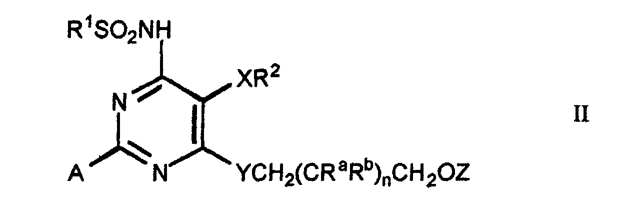
wherein A represents formyl or furan-2-yl and R¹, R², X, Y, Z, R^{a}, R^{b} and n have the significance given in daim 1 and wherein a hydroxy group represented in formula II by OZ and a hydroxy group which may be present as R^{a} and/or in R¹ can be present in protected form as an ester or ketal,
into a carboxyl group, optionally converting the hydroxy group R³ in a thus-obtained compound of formula I in which R³ is hydroxy into a lower-alkoxy group or a group -NR⁴R⁵, if necessary converting an esterified or ketalized hydroxy group into a free hydroxy group and optionally converting a compound of formula I into a pharmaceutically usable salt.

## Revendications

1. Composés de formule I 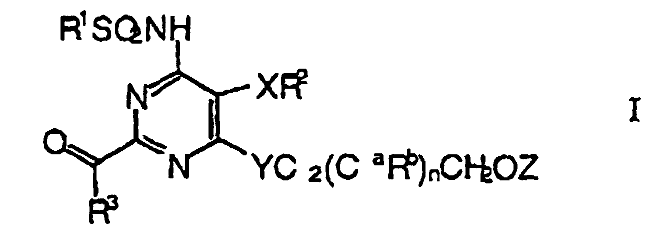 dans laquelle
R¹ représente le groupe phényle, un groupe phényle ou hétérocyclyle substitué par un groupe alkyle Inférieur, alcoxy inférieur, méthylènedioxy, éthylènedioxy, alcanoyle inférieur, hydroxy, amino, mono- ou di(alkyl inférieur)amino et/ou par un atome d'halogène ;
R² représente le groupe phényle ou un groupe phényle substitué par un groupe alkyle inférieur, alcoxy inférieur, méthylènedioxy, éthylènedioxy, alcanoyle Inférieur, hydroxy, amino, mono- ou di(alkyl Inférieur)amine et/ou par un atome d'halogène;
R³ représente un groupe hydroxy, alcoxy inférieur ou un radical -NR⁴R⁵;
R⁴ représente un atome d'hydrogène ou un radical -R⁶ ;
R⁵ représente un atome d'hydrogène ou un radical -(CH₂)ₘR⁶, ou
R⁴ et R⁵ forment ensemble, avec l'atome d'azote qui les relie, un radical hétérocyclyle monocyclique à 6 chaînons, qui peut comporter un autre atome d'oxygène ou d'azote et être substitué sur ce dernier atome d'azote par un groupe alkyle inférieur ;
R⁶ représente le groupe phényle, un groupe phényfe substitué par un groupe alkyle Inférieur, alcoxy inférieur, méthylènedioxy, éthylènedioxy, alcanoyle inférieur, hydroxy, amino, mono- ou di(alkyl inférieur)amino et/ou par un atome d'halogène ; un groupe cycloalkyle en C₃-C₈, hétérocyclyle, alkyle inférieur, hydroxyalkyle inférieur, aminoalkyle Inférieur, carboxyalkyle inférieur ou (alcoxy Inférieur)carbonyl-alkyle Inférieur ;
R^{a} représente un atome d'hydrogène, un groupe alkyle Inférieur ou hydroxy ;
R^{b} représente un atome d'hydrogène ou un groupe alkyle inférieur;
X représente un atome d'oxygène ou de soufre ;
Y représente un atome d'oxygène ou de soufre ;
Z représente un atome d'hydrogène, un groupe alkyle inférieur, aryle, arylalkyle inférieur, hétérocyclyle ou hétérocyclyl-alkyle inférieur ;
m est 0, 1 ou 2 ;
n est 0, 1 ou 2;
"hétérocyclyle" signifie un radical hétérocyclique mono- ou bicyclique à S ou 6 chaînons, comportant un atome d'oxygène, d'azote ou de soufre en tant qu'hétéroatome, et éventuellement substitué par un groupe alkyle inférieur, alcanoyle Inférieur, hydroxy, alcanoyloxy Inférieur, alcoxy inférieur, alcoxycarbonyle inférieur, formyle, amino, mono- ou di(alkyl inférieur)amino ou par un atome d'halogène ;
"aryle" signifie un groupe phényle éventuellement substitué par un groupe alkyle inférieur, alcoxy inférieur, méthylènedioxy, éthylènedioxy, alcanoyle inférieur, hydroxy, amino, mono- ou di(alkyl inférieur)amino et/ou par un atome d'halogène ;
et "inférieur" signifie des groupes ayant de 1 à 7 atomes de carbone;
et leurs sels pharmaceutiquement utilisables.

2. Composés selon la revendication 1, dans lesquels X et Y représentent des atomes d'oxygène.

3. Composés selon la revendication 1 ou 2, dans lesquels n est 0 ou 1.

4. Composés selon l'une quelconque des revendications 1 à 3, dans lesquels R¹ peut être remplacé par un groupe phényle substitué par un groupe alkyle inférieur ou alcoxy inférieur.

5. Composés selon l'une quelconque des revendications 1 à 3, dans lesquels R¹ représente un radical hétérocyclyle monocydique contenant un atome d'azote, et éventuellement substitué par un groupe alkyle inférieur.

6. Composés selon l'une quelconque des revendications 1 à 5, dans lesquels R² représente un groupe phényle substitué par un groupe alcoxy et/ou par un atome d'halogène.

7. Composés selon l'une quelconque des revendications 1 à 3, dans lesquels R³ représente un groupe hydroxy ou NR⁴R⁵.

8. Composés selon l'une quelconque des revendications 1 à 6, dans lesquels R³ est un groupe NR⁴R⁵, R⁵ est un groupe -(CH₂)ₘR⁶ et R⁶ est un groupe phényle, phényle substitué, cycloalkyle en C₃₋₈ ou hétérocyclyle.

9. Les composés selon la revendication 4,
acide 6-(4-tert-butyl-phénylsulfonylamino)-4-(2-hydroxy-éthoxy)-5-(3-méthoxy-phénoxy)-pyrimidine-2-carboxylique,
acide 4-(4-tert-butyl-phénylsulfonylamino)-5-(2-chloro-5-méthoxyphénoxy)-6-(2-hydroxy-éthoxy)-pyrimidine-2-carboxylique,
acide 4-(2-hydroxy-éthoxy)-5-(3-méthoxy-phénoxy)-6-(4-méthoxy-phénylsulfonylamino)-pyrimidine-2-carboxylique,
acide (RS)-6-(4-tert-butyl-phénylsulfonylamino)-4-(2,3-dihydroxypropoxy)-5-(3-méthoxy-phénoxy)-pyrimidine-Z-carboxylique,
acide 6-(4-tert-butyl-phénylsulfonylamino)-4-(2-hydroxy-éthoxy)-5-(2-méthoxy-phénoxy)-pyrimidine-2-carboxylipue,
6-(4-tert-butyl-phénylsulfonylamino)-4-(2-hydroxy-éthoxy)-5-(3-méthoxyphénoxy)-pyrimidine-2-phényl-carboxamide,
4-(2-hydroxy-éthoxy)-5-(3-méthoxy-phénoxy)-6-(4-méthoxy-phénylsulfonylamino)-pyrimidine-2-phényl-carboxamide,
6-(4-tert-butyl-phénylsulfonylamino)-5-(2-méthoxy-phénoxy)-4-(2-hydroxy-éthoxy)-pyrimidine-2-phényl-carboxamide,
4-(4-tert-butyl-phénylsulfonylamino)-5-(2-chloro-5-méthoxy-phénoxy)-6-(2-hydrox-éthoxy)-pyrimidine-2-phényl-carboxamide,
4-(4-tert-butyl-phénylsulfonylamino)-6-(2-hydroxy-éthoxy)-5-(3-méthoxyphénoxy)-pyrimidine-2-(1H-tétrazol-5-yl)-carboxamide.

10. Les composés sélon la revendication 5,
acide 4-(2-hydroxy-éthoxy)-6-(5-isopropyl-pyrldin-2-ylsulfonylamino)-5-(3-méthoxy-phénoxy)-pyrimidine-2-carboxylique,
acide 4-(2-hydroxy-éthoxy)-6-(5-isopropyl-pyridin-2-ylsulfonylamino)-5-(3-méthoxy-phénoxy)-pyrimidine-2-carboxylique.

11. Composés de formule 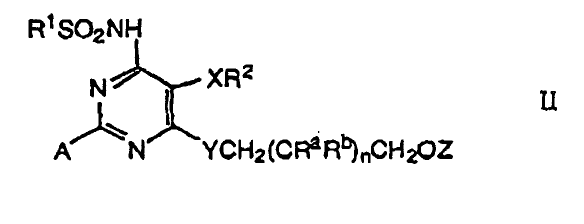
dans laquelle A représente le groupe formyle ou furann-2-yle et R¹, R², X, Z, R^{a}, R^{b}, Y et n ont les significations données dans la revendication 1 et un groupe hydroxy représenté par OZ dans la formule II et un groupe hydroxy éventuellement présent en tant que R^{a} et/ou dans R¹ pouvant être protégés sous forme d'ester ou de cétal.

12. Composés des revendications 1-10 pour utilisation en tant que médicament.

13. Compositions pharmaceutiques contenant un composé des revendications 1-10 et des véhicules et adjuvants usuels.

14. Utilisation des composés des revendications 1-10 en tant que substances actives dans la fabrication de médicaments destinés au traitement de maladies qui sont associées à des activités de l'endothéline, en particulier de maladies circulatoires telles que l'hypertonie, l'ischémle, les angiospasmes et l'angine de poitrine.

15. Procédé pour la préparation de composés des revendications 1-10, **caractérisé en ce que** dans un composé de formule II 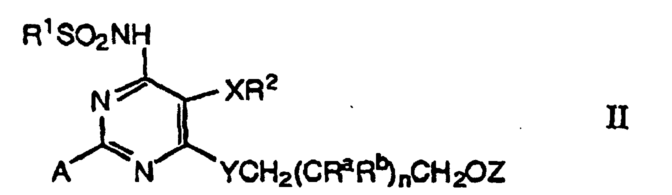
dans laquelle A représente le groupe formyle ou furann-2-yle et R¹, R², X, Y, Z, R^{a}, R^{b} et n ont les signiflcations données dans la revendication 1 et un groupe hydroxy représenté par OZ dans la formule II et un groupe hydroxy éventuellement présent en tant que R^{a} et/ou dans R¹ pouvant être protégés sous forme d'ester ou de cétal,
on convertit le radical A en un groupe carboxy, éventuellement dans le composé de formule I ainsi obtenu, dans lequel R³ est un groupe hydroxy, on convertit le groupe hydroxy R³ en un groupe alcoxy inférieur ou en un groupe -NR⁴R⁵, si nécessaire on convertit un groupe hydroxy estérifié ou cétalisé en un groupe hydroxy libre et éventuellement on convertit un composé de formule I en un sel pharmaceutiquement utilisable.
